(19) Europäisches Patentamt
European Patent Office
Office européen des brevets

(11) EP 2 218 719 A1

(12) EUROPEAN PATENT APPLICATION
published in accordance with Art. 153(4) EPC

(43) Date of publication:
**18.08.2010 Bulletin 2010/33**

(21) Application number: **08856722.7**

(22) Date of filing: **02.12.2008**

(51) Int Cl.:
***C07D 409/04*** *(2006.01)* ***A61K 31/4535*** *(2006.01)*
***A61K 31/454*** *(2006.01)* ***A61K 31/4545*** *(2006.01)*
***A61K 31/496*** *(2006.01)* ***A61K 31/5377*** *(2006.01)*
***A61K 31/55*** *(2006.01)* ***A61P 35/00*** *(2006.01)*
***A61P 43/00*** *(2006.01)* ***C07D 409/14*** *(2006.01)*

(86) International application number:
**PCT/JP2008/003557**

(87) International publication number:
**WO 2009/072267 (11.06.2009 Gazette 2009/24)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **03.12.2007 JP 2007312176**

(71) Applicant: **Kabushiki Kaisha Yakult Honsha**
**Minato-ku**
**Tokyo 1058860 (JP)**

(72) Inventors:
- **YAMAZAKI, Ryuta**
  **Tokyo 105-8660 (JP)**
- **HATANO, Hiroshi**
  **Tokyo 105-8660 (JP)**
- **YAEGASHI, Takashi**
  **Tokyo 105-8660 (JP)**
- **IGARASHI, Yoshiaki**
  **Tokyo 105-8660 (JP)**
- **YOSHIDA, Oh**
  **Tokyo 105-8660 (JP)**
- **SUGIMOTO, Yoshikazu**
  **Kashiwa-shi**
  **Chiba 277-0061 (JP)**

(74) Representative: **Blodig, Wolfgang**
**Wächtershäuser & Hartz**
**Patentanwälte**
**Weinstraße 8**
**D-80333 München (DE)**

# (54) ABCG2 INHIBITOR

(57) The present invention relates to a breast cancer resistance protein (BCRP/ABCG2) inhibitor.
The invention provides an acrylonitrile derivative represented by formula (1):

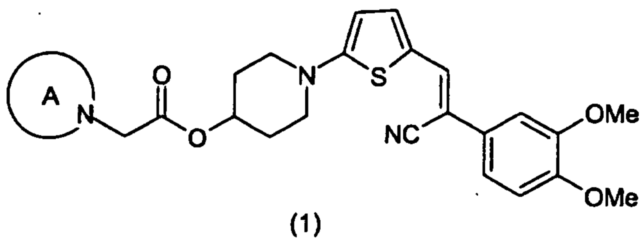

(1) (E or Z)

(wherein A represents an optionally substituted 3- to 8-membered heterocyclic ring) or a salt thereof.

EP 2 218 719 A1

Printed by Jouve, 75001 PARIS (FR)

## Description

### Technical Field

The present invention relates to a breast cancer resistance protein (BCRP/ABCG2) inhibitor.

### Background Art

Serious problems associated with cancer chemotherapy include intrinsic resistance to an anticancer agent, which invalidates the effect of the anticancer agent from the beginning of cancer therapy, and development of acquired resistance to an anticancer agent (i.e., reduction of the effect of the drug, which is caused by long-term continuous administration thereof). Overcoming such resistance to anticancer agents has been envisaged to lead to improvement in the performance of cancer chemotherapy, and thus attempts have been made to elucidate various resistance mechanisms. In particular, expression of a drug transport protein, which actively transports an anticancer agent out of cancer cells, thereby reducing the amount of intracellular accumulation of the drug, is considered to play an important role in such a resistance mechanism.
P-glycoprotein, in particular, which is a drug transport protein discovered in the 1970s and is encoded by an MDR1 gene, has been considered a potent target molecule of a multidrug-resistance-overcoming agent, since this protein causes cross-resistance to a plurality of anticancer agents having different chemical structures and action mechanisms. However, it has been gradually elucidated that the anticancer agent resistance mechanism cannot be analyzed on the sole basis of P-glycoprotein, and demand has arisen for development of a resistance-overcoming agent which targets another drug transport protein.
Under such circumstances, there was discovered, in 1998, breast cancer resistance protein (BCRP, also called ABCG2, MXR, or ABCP), which is a drug transport protein belonging to a group which is called "ATP-binding cassette (ABC) transporter superfamily" to which P-glycoprotein also belongs (see Non-Patent Document 1). BCRP has a structure including only one ATP-binding cassette, which differs from that of P-glycoprotein or another ABC transporter, which has two ATP-binding cassettes. BCRP is involved in the mechanism of resistance to a topoisomerase I inhibitor (e.g., irinotecan hydrochloride (CPT-11) or topotecan), to a topoisomerase II inhibitor (e.g., mitoxantrone), or to a molecule-targeting therapeutic drug (e.g., gefitinib and imatinib). Meanwhile, BCRP has been elucidated to exhibit substrate specificity different from that of P-glycoprotein, since BCRP does not act on, for example, paclitaxel or vincristine, which is excreted by P-glycoprotein, and BCRP is involved in excretion of a camptothecin derivative (e.g., CPT-11 or 7-ethyl-10-hydroxycamptothecin (SN-38: active metabolite of CPT-11), which is barely excreted extracellularly by P-glycoprotein (see Non-Patent Document 2). In addition, BCRP has been suggested to be involved in the limitation of the bioavailability of an orally administered anticancer agent (see Non-Patent document 3). In view of the foregoing, demand has arisen for development of a BCRP inhibitor, which is envisaged to exhibit the effect of overcoming anticancer agent resistance that is not overcome by a conventional resistance-overcoming agent, and to improve the bioavailability of an anticancer agent.
Hitherto, in an attempt to overcome resistance to anticancer agents, a variety of P-glycoprotein inhibitors have been developed. In contrast, only a few reports have been given for BCRP inhibitors, and the reported inhibitory action is not satisfactory. Therefore, continuous efforts have been made to develop more effective BCRP inhibitors. Examples of the compounds exhibiting BCRP inhibitory action which have heretofore reported include an FTC (Fumitremorgin C) derivative (see Non-Patent Document 4), estrogen and anti-estorgen (see Non-Patent Document 5), and novobiocin (see Non-Patent Document 6). The present inventors also found that a flavonoid (see Patent Document 1), a diphenylacrylonitrile derivative (see Patent Document 2), and an acrylonitrile derivative having a heterocyclic ring (see Patent Document 3) have BCRP inhibitory action. However, the effects of these compounds are unsatisfactory, and problems in use as pharmaceuticals such as solubility and toxicity have not yet been solved.

Patent Document 1: WO 2004/069233, pamphlet
Patent Document 2: WO 2004/069243, pamphlet
Patent Document 3: WO 2006/106778, pamphlet
Non-Patent Document 1: Proc. Natl. Acad. Sci. USA, 1998, 95: 15665-15670
Non-Patent Document 2: Cancer Res., 1999, 59: 5938-5946
Non-Patent Document 3: J. Clin. Oncol., 2002, 20: 2943-2950
Non-Patent Document 4: Mol. Cancer Ther., 2002, 1: 417-425
Non-Patent Document 5: Mol. Cancer Ther., 2003, 2: 105-112
Non-Patent Document 6: Int. J. Cancer, 2004, 108: 146-151

Disclosure of the Invention

Problems to be Solved by the Invention

An object of the present invention is to provide a novel drug which has excellent breast cancer resistance protein (BCRP) inhibitory action and improved solubility. Means for Solving the Problems

In an attempt to solve the aforementioned problems, the present inventors have carried out screening of compounds by use of cancer cells which have acquired anticancer drug resistance through BCRP expression, and have found that acrylonitrile derivatives represented by the following formula (1) exhibit potent BCRP inhibitory action.

Accordingly, the present invention provides an acrylonitrile derivative represented by formula (1):

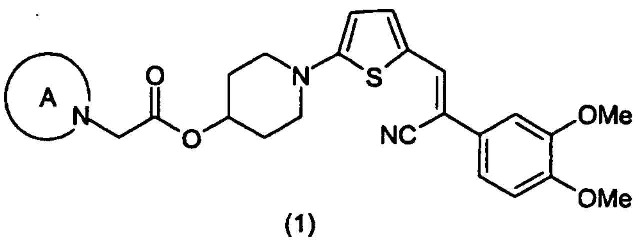

(1) (E or Z)

(wherein A represents an optionally substituted 3- to 8-membered heterocyclic ring) or a salt thereof.

The present invention also provides a drug containing, as an active ingredient, the acrylonitrile derivative or a salt thereof as described above.

The present invention also provides a BCRP inhibitor containing, as an active ingredient, the acrylonitrile derivative or a salt thereof as described above.

The present invention also provides an agent for overcoming anticancer agent resistance or an agent for potentiating anticancer agent effect, which agent contains, as an active ingredient, the acrylonitrile derivative or a salt thereof as described above.

The present invention also provides a pharmaceutical composition comprising the acrylonitrile derivative or a salt thereof as described above and a pharmaceutically acceptable carrier.

The present invention also provides an anticancer agent composition comprising the acrylonitrile derivative or a salt thereof as described above and an anticancer agent which serves as a BCRP substrate.

The present invention also provides use of the acrylonitrile derivative or a salt thereof as described above for producing an agent for overcoming anticancer agent resistance or an agent for potentiating anticancer agent effect.

The present invention also provides use of the acrylonitrile derivative or a salt thereof as described above for producing an anticancer agent composition comprising an anticancer agent which serves as a BCRP substrate.

The present invention also provides a method for treatment of cancer which has acquired drug resistance by the mediation of BCRP, the method comprising administering, to a subject in need thereof, the acrylonitrile derivative or a salt thereof as described above.

The present invention also provides a method for inhibiting BCRP, the method comprising administering, to a subject in need thereof, the acrylonitrile derivative or a salt thereof as described above.

The present invention also provides a method for overcoming anticancer agent resistance or for potentiating anticancer agent effect, the method comprising administering, to a subject in need thereof, the acrylonitrile derivative or a salt thereof as described above.

The present invention also provides a method for treatment of cancer, the method comprising administering, to a subject in need thereof, the acrylonitrile derivative or a salt thereof as described above and an anticancer agent which serves as a BCRP substrate.

Effects of the Invention

According to the present invention, the BCRP inhibitory effect of the acrylonitrile derivative or a salt thereof can overcome BCRP-related resistance to an anticancer agent. In addition, the effect of an anticancer agent with respect to cancer cells in which BCRP is intrinsically expressed can be potentiated. Furthermore, according to the present invention, bioavailability of an anticancer agent is envisaged to be enhanced, leading to improvement in the performance of cancer chemotherapy.

The acrylonitrile derivative or a salt thereof of the present invention, which has excellent solubility, is a very useful raw material for producing drugs.

Best Modes for Carrying Out the Invention

In formula (1), examples of the heterocyclic ring A include 3- to 8-membered heterocyclic rings. Of these, 4- to 8-membered heterocyclic rings are preferred. Specific examples of the heterocyclic ring A include aziridine, azetidine, pyrrolidine, piperidine, piperazine, thiomorpholine, morpholine, azepane, and azocane. Among them, the heterocyclic rings represented by formulas (2) to (9) are preferred, with piperidine represented by formula (5) being particularly preferred.
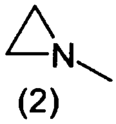
(2)

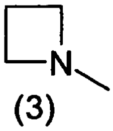
(3)

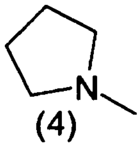
(4)

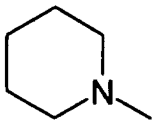
(5)

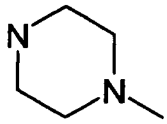
(6)

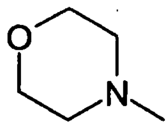
(7)

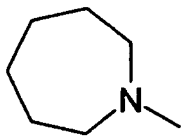
(8)

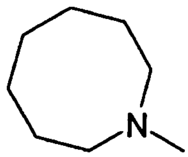
(9)

In formula (1), the heterocyclic ring A may further have one or more substituents. Examples of such substituents include a hydroxyl group, a lower alkyl group, a lower alkoxy group, a lower acyloxy group, a nitro group, an amino group, a halogen atom, a lower hydroxyalkyl group, and a lower alkoxycarbonyl group. When a plurality of substituents are present, those may be identical to or different from one another. Among them, heterocyclic rings having a hydroxyl group are particularly preferred.
Examples of the lower alkyl group include C1 to C6 alkyl groups. Specific examples include methyl, ethyl, n-propyl, and isobutyl.
Examples of the lower alkoxy group include C1 to C6 alkoxy groups. Specific examples include methoxy and ethoxy.
Examples of the lower acyloxy group include C1 to C6 acyloxy groups. Specific examples include formyloxy, acetoxy, and propionyloxy.
Examples of the lower hydroxyalkyl group include C1 to C6 hydroxyalkyl groups. Specific examples include hydroxymethyl and hydroxyethyl.
Examples of the lower alkoxycarbonyl group include C1 to C6 alkoxycarbonyl groups. Specific examples include methoxycarbonyl and ethoxycarbonyl.
Examples of the halogen atom include chlorine, bromine, fluorine, and iodine.
The acrylonitrile derivatives of the present invention may form pharmaceutically acceptable salts thereof, and these salts also fall within the scope of the present invention. Examples of the salts include inorganic salts such as hydrochlorides, sulfates, nitrates, and phosphates; alkali metal salts such as sodium salts and potassium salts; alkaline earth metal salts such as calcium salts and magnesium salts; and organic acid salts such as p-toluenesulfonates, methanesulfonates, fumarates, succinates, and lactates. The compounds of the present invention maybe present in the form of solvate (hydrate). The hydrates also fall within the scope of the present invention. The acrylonitrile derivatives of the present invention may include isomers thereof, and each of these isomers and mixtures of the isomers also fall within the scope of the present invention.
Of these, particularly preferred are the following compounds:

piperidin-1-yl-acetic acid 1-[5-[(Z)-2-cyano-2-(3,4-dimethoxy-phenyl)-vinyl]-thiophen-2-yl]-piperidin-4-yl ester,
(4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester,
azetidin-1-yl-acetic acid 1-[5-[(Z)-2-cyano-2-(3,4-dimethoxy-phenyl)-vinyl]-thiophen-2-yl]-piperidin-4-yl ester,
pyrrolidin-1-yl-acetic acid 1-[5-[(Z)-2-cyano-2-(3,4-dimethoxy-phenyl)-vinyl]-thiophen-2-yl]-piperidin-4-yl ester,
(2-methyl-piperidin-1-yl)-acetic acid 1-[5-[(Z)-2-cyano-2-(3,4-dimethoxy-phenyl)-vinyl]-thiophen-2-yl]-piperidin-4-yl ester,
(3-hydroxymethyl-piperidin-1-yl)-acetic acid 1-[5-[(Z)-2-cyano-2-(3,4-dimethoxy-phenyl)-vinyl]-thiophen-2-yl]-piperidin-4-yl ester,
azepan-1-yl-acetic acid 1-[5-[(Z)-2-cyano-2-(3,4-dimethoxy-phenyl)-vinyl]-thiophen-2-yl]-piperidin-4-yl ester,
azocan-1-yl-acetic acid 1-[5-[(Z)-2-cyano-2-(3,4-dimethoxy-phenyl)-vinyl]-thiophen-2-yl]-piperidin-4-yl ester,
(4-methyl-piperazin-1-yl)-acetic acid 1-[5-[(Z)-2-cyano-2-(3,4-dimethoxy-phenyl)-vinyl]-thiophen-2-yl]-piperidin-4-yl ester, and
morpholin-4-yl-acetic acid 1-[5-[(Z)-2-cyano-2-(3,4-dimethoxy-phenyl)-vinyl]-thiophen-2-yl]-piperidin-4-yl ester, and salts thereof.

The acrylonitrile derivatives of the present invention and salts thereof may be produced through, for example, the following reaction scheme (A).
Scheme (A)
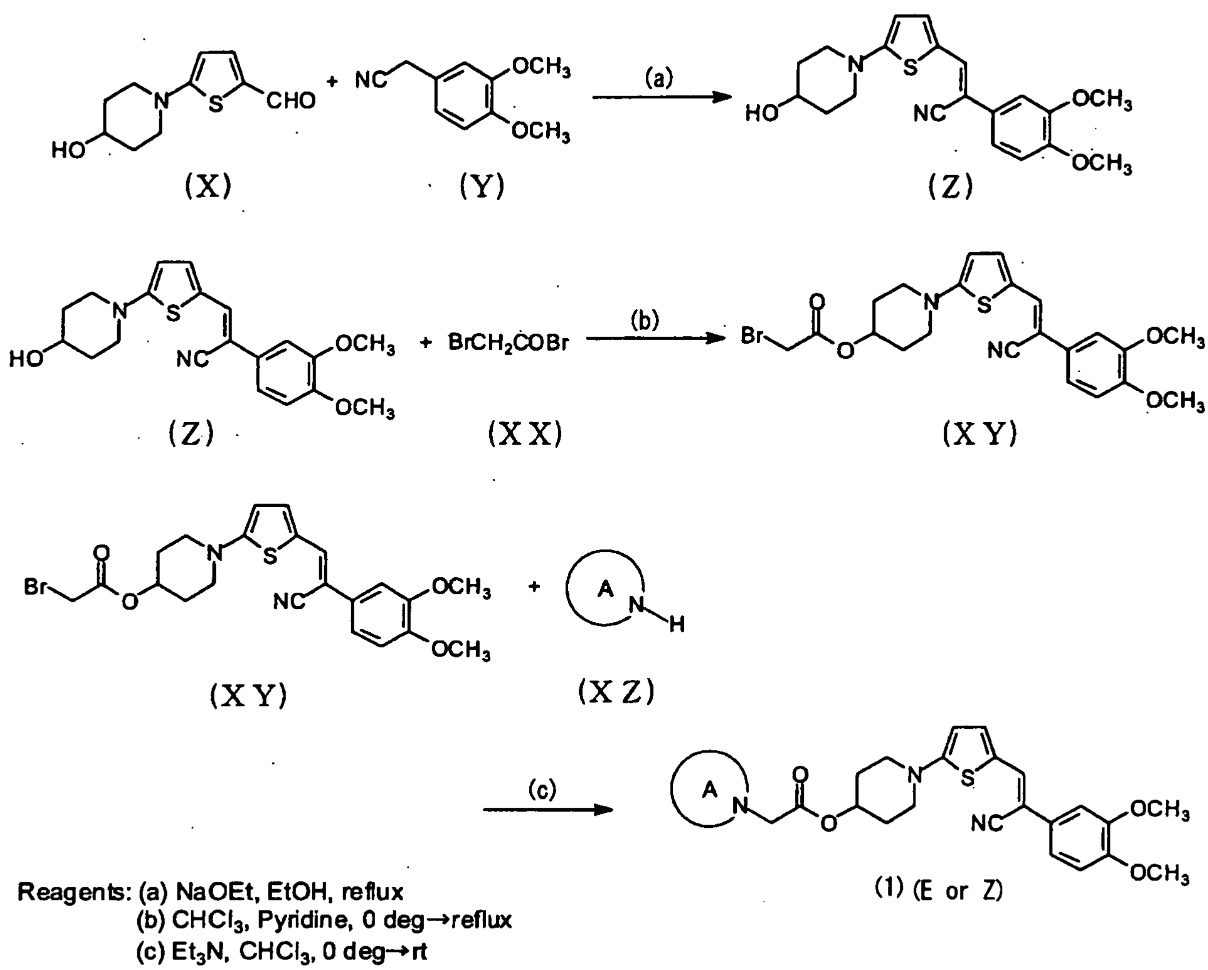


Specifically, through condensation between 5-(4-hydroxypiperidin-1-yl)-thiophene-2-carboxaldehyde (X) and 3,4-dimethoxybenzyl cyanide (Y), (Z)-2-(3,4-dimethoxy-phenyl)-3-[5-(4-hydroxy-piperidin-1-yl)-thiophen-2-yl]-acrylonitrile (Z) is produced.
Further reaction of (Z) with bromoacetyl bromide (XX) yields bromo-acetic acid 1-[5-[(Z)-2-cyano-2-(3,4-dimethoxy-phenyl)-vinyl]-thiophen-2-yl]-piperidin-4-yl ester (XY). Reaction of bromo-acetic acid 1-[5-[(Z)-2-cyano-2-(3,4-dimethoxy-phenyl)-vinyl]-thiophen-2-yl]-piperidin-4-yl ester (XY) with a secondary amine of interest (XZ, wherein A has the same meaning as defined above) yields an acrylonitrile derivative of the present invention represented by formula (1) (wherein A has the same meaning as defined above).

No particular limitation is imposed on the secondary amine (XZ, wherein A has the same meaning as defined above). Specific examples thereof include piperidine, 4-hydroxypiperidine, aziridine-2-carboxylic acid methyl ester, azetidine, pyrrolidine, 2-methyl-piperidine, 3-hydroxymethyl-piperidine, azepane (hexamethyleneimine), azocane (heptamethyleneimine), 4-methyl-piperazine, morpholine, and thiomorpholine. Examples of the acrylonitrile derivative produced by use of the secondary amine in the above reaction scheme (A) include:

piperidin-1-yl-acetic acid 1-[5-[(Z)-2-cyano-2-(3,4-dimethoxy-phenyl)-vinyl]-thiophen-2-yl]-piperidin-4-yl ester,
(4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester,
1-(1-[5-[(Z)-2-cyano-2-(3,4-dimethoxy-phenyl)-vinyl]-thiophen-2-yl]-piperidin-4-yloxycarbonylmethyl)-aziridine-2-carboxylic acid methyl ester,
azetidin-1-yl-acetic acid 1-[5-[(Z)-2-cyano-2-(3,4-dimethoxy-phenyl)-vinyl]-thiophen-2-yl]-piperidin-4-yl ester,
pyrrolidin-1-yl-acetic acid 1-[5-[(Z)-2-cyano-2-(3,4-dimethoxy-phenyl)-vinyl]-thiophen-2-yl]-piperidin-4-yl ester,
(2-methyl-piperidin-1-yl)-acetic acid 1-[5-[(Z)-2-cyano-2-(3,4-dimethoxy-phenyl)-vinyl]-thiophen-2-yl]-piperidin-4-yl ester,
(3-hydroxymethyl-piperidin-1-yl)-acetic acid 1-[5-[(Z)-2-cyano-2-(3,4-dimethoxy-phenyl)-vinyl]-thiophen-2-yl]-piperidin-4-yl ester,
azepan-1-yl-acetic acid 1-[5-[(Z)-2-cyano-2-(3,4-dimethoxy-phenyl)-vinyl]-thiophen-2-yl]-piperidin-4-yl ester,
azocan-1-yl-acetic acid 1-[5-[(Z)-2-cyano-2-(3,4-dimethoxy-phenyl)-vinyl]-thiophen-2-yl]-piperidin-4-yl ester,
(4-methyl-piperazin-1-yl)-acetic acid 1-[5-[(Z)-2-cyano-2-(3,4-dimethoxy-phenyl)-vinyl]-thiophen-2-yl]-piperidin-4-yl ester,
morpholin-4-yl-acetic acid 1-[5-[(Z)-2-cyano-2-(3,4-dimethoxy-phenyl)-vinyl]-thiophen-2-yl]-piperidin-4-yl ester, and
thiomorpholin-4-yl-acetic acid 1-[5-[(Z)-2-cyano-2-(3,4-dimethoxy-phenyl)-vinyl]-thiophen-2-yl]-piperidin-4-yl ester.

The condensation reaction is preferably carried out in the presence of a base such as sodium alkoxide, sodium hydroxide, or potassium hydroxide. When sodium alkoxide is employed, the condensation reaction is performed in an alcoholic solvent, such as methanol or ethanol, at between ice cooling temperature and reflux temperature, whereas when sodium hydroxide is employed, the condensation reaction is performed in a solvent mixture of water and an inert solvent, such as methylene chloride or chloroform, with a quaternary ammonium salt or a similar compound being added thereto.

The acrylonitrile derivatives each having a heterocyclic ring of the present invention or salts thereof may be administered as is. Alternatively, the derivatives or salts thereof may be mixed with a pharmaceutically acceptable carrier such as a dispersing aid or an excipient, and may be used in the form of an injection or a peroral preparation such as powder, solution, capsules, suspension, emulsion, syrup, elixir, granules, pills, tablets, troches, or lemonade. These products may be prepared through a conventional method.

Examples of such a carrier include water-soluble monosaccharides, oligosaccharides, and polysaccharides, such as mannitol, lactose, and dextran; gel-forming or water-soluble celluloses, such as hydroxypropyl cellulose, hydroxypropylmethyl cellulose, and methyl cellulose; water-absorbing and poorly water-soluble celluloses, such as crystalline cellulose, $\alpha$-cellulose, cross-linked carboxymethylcellulose sodium, and derivatives thereof; water-absorbing and poorly water-soluble polysaccharides, such as hydroxypropyl starch, carboxymethyl starch, cross-linked starch, amylose, amylopectin, pectin, and derivatives thereof; water-absorbing and poorly water-soluble gums, such as gum arabic, tragacanth gum, glucomannan, and derivatives thereof; cross-linked vinyl polymers, such as polyvinyl pyrrolidone, cross-linked polyacrylic acid and salts thereof, cross-linked polyvinyl alcohol, polyhydroxyethyl methacrylate, and derivatives thereof; and molecular aggregate (e.g., liposome)-forming lipids, such as phospholipid and cholesterol.

The solubility of the compound of the present invention may further be enhanced through solubilization. Examples of the solubilization technique include techniques which are generally applicable to drugs, such as a technique in which a surfactant (e.g., a polyoxyethylene alcohol ether, a polyoxyethylene acyl ester, a sorbitan acyl ester, or a polyoxyethylene sorbitan acyl ester) is added, and a technique employing a water-soluble polymer (e.g., polyethylene glycol). If desired, there may be employed, for example, a technique for forming a soluble salt of the compound, or a technique for forming a clathrate compound by use of cyclodextrin or a similar material. A solubilization technique may be appropriately selected in accordance with the target acrylonitrile derivative or a salt thereof.

By virtue of a potent BCRP inhibitory effect, the compound of the present invention can be employed as a BCRP inhibitor, an agent for overcoming anticancer agent resistance, and an agent for potentiating anticancer agent effect. The compound of the invention can also be used in an anticancer agent composition in combination with another anticancer agent which serves as a BCRP substrate. The compound may be employed as an agent for overcoming

anticancer agent resistance for a cancer which has acquired BCRP-associated resistance through administration of an anticancer drug. Meanwhile, the compound may be employed as an agent for potentiating anticancer agent effect for a cancer which originally expresses BCRP and exhibits low sensitivity to an anticancer drug. No particular limitation is imposed on the target anticancer drug on which the agent for overcoming anticancer agent resistance or agent for potentiating anticancer agent effect containing, as an active ingredient, the BCRP inhibitor of the present invention, acts, so long as the anticancer drug can serve as a substrate for BCRP or an analog thereof. Examples of such an anticancer drug include topoisomerase I inhibitors such as irinotecan hydrochloride/CPT-11 (active form: SN-38) and topotecan; topoisomerase II inhibitors such as mitoxantrone, doxorubicin, daunorubicin, bisanthrene, and etoposide; antifolates such as methotrexate; and molecule-targeting therapeutic drugs such as gefitinib and imatinib. Notably, no particular limitation is imposed on the BCRP analog, so long as it has the same actions on anticancer resistance and sensitivity to anticancer agents as those of BCRP.

The dose of the BCRP inhibitor of the present invention may be appropriately determined in accordance with, for example, the administration method or the symptom of a patient. The daily dose for an adult is preferably 1 mg to 10 g, more preferably 100 mg to 10 g, particularly preferably 500 mg to 10 g. No particular limitation is imposed on the ratio between an anticancer drug and the BCRP inhibitor, and the preferred ratio varies in accordance with, for example, the type of an anticancer drug or inhibitor to be employed. When, for example, irinotecan hydrochloride is employed as an anticancer drug, the ratio by weight of the anticancer drug to the BCRP inhibitor is preferably 1:1 to 1:500, particularly preferably 1:1 to 1:100, more preferably 1:1 to 1:10.

## Examples

The present invention will next be described in more detail by way of examples, which should not be construed as limiting the invention thereto.

Example 1: Production of an acrylonitrile derivative having a heterocyclic ring

Production Step 1: Step of producing 5-(4-hydroxypiperidin-1-yl)-thiophene-2-carboxaldehyde (step of incorporating 4-hydroxypiperidine into 5-bromothiophene-2-carboxaldehyde)

5-Bromothiophene-2-carboxaldehyde was placed in a reactor, and water was added thereto. 4-Hydroxypiperidine (3 eq) was added to the reactor, and the mixture was stirred for tens of minutes or overnight under reflux. Immediately after completion of reaction, the reaction mixture was filtered through filter paper, and the filtrate was cooled for tens of minutes with a flow of water and then for several hours with ice. The precipitated crystals were recovered through filtration under suction and washed with cold water. The crystals were dried and dissolved in chloroform. The chloroform solution was dried over sodium sulfate anhydrate, and the dried solution was filtered through a silica gel pad. The filtrate was washed with chloroform until the color thereof became faint. The filtrate was concentrated under reduced pressure until the start of crystallization. n-Hexane was added to the mixture, and stirring was performed overnight at room temperature. The formed crystals were recovered through filtration, washed with n-hexane, and dried under reduced pressure, to thereby yield 5-(4-hydroxypiperidin-1-yl)-thiophene-2-carboxaldehyde.

Production Step 2: Step of producing (Z)-2-(3,4-dimethoxy-phenyl)-3-[5-(4-hydroxy-piperidin-1-yl)-thiophen-2-yl]-acrylonitrile (step of condensation between 5-(4-hydroxypiperidin-1-yl)-thiophene-2-carboxaldehyde and 3,4-dimethoxybenzyl cyanide)

5-(4-Hydroxypiperidin-1-yl)-thiophene-2-carboxaldehyde and 3,4-dimethoxybenzyl cyanide were placed in a reactor and dissolved in ethanol. Sodium ethoxide was added thereto, and the mixture was stirred under reflux. After completion of reaction, the reaction mixture was cooled for tens of minutes with a flow of water. Water was added to the cooled mixture, and stirring was performed for tens of minutes. The precipitated crystals were recovered through filtration and washed sequentially with water, ethanol, and hexane, followed by drying under reduced pressure, to thereby yield (Z)-2-(3,4-dimethoxy-phenyl)-3-[5-(4-hydroxy-piperidin-1-yl)-thiophen-2-yl]-acrylonitrile.

Production Step 3 (Method A): Step of producing bromo-acetic acid 1-[5-[(Z)-2-cyano-2-(3,4-dimethoxy-phenyl)-vinyl]-thiophen-2-yl]-piperidin-4-yl ester

Under argon, (Z)-2-(3,4-dimethoxy-phenyl)-3-[5-(4-hydroxy-piperidin-1-yl)-thiophen-2-yl]-acrylonitrile was placed in a reactor and dissolved in chloroform. Pyridine was added to the solution, and the mixture was stirred in an ice bath for tens of minutes. Bromoacetyl bromide was added to the reaction mixture, and stirring was performed in an ice bath for several hours and overnight at room temperature. After completion of reaction, chloroform and water were added to the reaction mixture for phase separation. The recovered organic layer was washed with water and dried over

magnesium sulfate anhydrate. The solvent was evaporated under reduced pressure. The residue was purified through silica gel column chromatography, to thereby yield bromo-acetic acid 1-[5-[(Z)-2-cyano-2-(3,4-dimethoxy-phenyl)-vinyl]-thiophen-2-yl]-piperidin-4-yl ester.

Production Step 3 (Method B): Step of producing bromo-acetic acid 1-[5-[(Z)-2-cyano-2-(3,4-dimethoxy-phenyl)-vinyl]-thiophen-2-yl]-piperidin-4-yl ester

(Z)-2-(3,4-dimethoxy-phenyl)-3-[5-(4-hydroxy-piperidin-1-yl)-thiophen-2-yl]-acrylonitrile was placed in a reactor and dissolved in tetrahydrofuran, followed by cooling with ice. Sodium hydride was added to the solution, and the mixture was stirred in an ice bath for tens of minutes. Bromoacetyl chloride was added to the reaction mixture, and stirring was performed for several hours under reflux. After allowing the mixture to stand for cooling, chloroform and water were added thereto for phase separation. The recovered organic layer was washed with water and dried over sodium sulfate anhydrate. The solvent was evaporated under reduced pressure. The residue was purified through silica gel column chromatography, to thereby yield bromo-acetic acid 1-[5-[(Z)-2-cyano-2-(3,4-dimethoxy-phenyl)-vinyl]-thiophen-2-yl]-piperidin-4-yl ester.

Production Step 4: Step of incorporating amine to bromo-acetic acid 1-[5-[(Z)-2-cyano-2-(3,4-dimethoxy-phenyl)-vinyl]-thiophen-2-yl]-piperidin-4-yl ester

Under argon, bromo-acetic acid 1-[5-[(Z)-2-cyano-2-(3,4-dimethoxy-phenyl)-vinyl]-thiophen-2-yl]-piperidin-4-yl ester was placed in a reactor and dissolved in chloroform. The mixture was stirred in an ice bath for tens of minutes. A secondary amine of interest and a tertiary amine of interest (e.g., triethylamine) were added thereto, and stirring was performed in an ice bath for several hours and overnight at room temperature. After completion of reaction, the reaction mixture was washed with water, and the organic layer was dried over sodium sulfate anhydrate. The solvent was evaporated under reduced pressure. The residue was purified through silica gel column chromatography, to thereby yield an acrylonitrile derivative of interest.

Specific examples of production of various derivatives and analytical results of the produced derivatives will next be described.

Production of piperidin-1-yl-acetic acid 1-[5-[(Z)-2-cyano-2-(3,4-dimethoxy-phenyl)-vinyl]-thiophen-2-yl]-piperidin-4-yl ester (Compound 1)

By use of 5-bromothiophene-2-carboxaldehyde (42.30 g) and 4-hydroxypiperidine (67.30 g), amine incorporation was performed according to Production Step 1, to thereby yield 5-(4-hydroxy-piperidin-1-yl)-thiophene-2-carboxaldehyde (yield: 33.00 g, 71%). The thus-produced 5-(4-hydroxy-piperidin-1-yl)-thiophene-2-carboxaldehyde (10.56 g) and 3,4-dimethoxybenzyl cyanide (8.86 g) were subjected to condensation according to Production Step 2, to thereby yield (Z)-2-(3,4-dimethoxy-phenyl)-3-[5-(4-hydroxy-piperidin-1-yl)-thiophen-2-yl]-acrylonitrile (yield: 13.50 g, 73%). The thus-produced (Z)-2-(3,4-dimethoxy-phenyl)-3-[5-(4-hydroxy-piperidin-1-yl)-thiophen-2-yl]-acrylonitrile (20.00 g) was dissolved in chloroform (650 mL), and the solution was reacted with pyridine (6.41 g) and bromoacetyl bromide (14.13 g) according to Production Step 3 (Method A), to thereby yield bromo-acetic acid 1-[5-[(Z)-2-cyano-2-(3,4-dimethoxy-phenyl)-vinyl]-thiophen-2-yl]-piperidin-4-yl ester (yield: 23.00 g, 87%). The thus-produced bromo-acetic acid 1-[5-[(Z)-2-cyano-2-(3,4-dimethoxy-phenyl)-vinyl]-thiophen-2-yl]-piperidin-4-yl ester (2.30 g) was dissolved in chloroform (100 mL), and the solution was reacted with piperidine (533 mg) and triethylamine (658 mg) according to Production Step 4, to thereby yield the title compound (yield: 1.40 g, 60%).

Production of (4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester (Compound 2)

Bromo-acetic acid 1-[5-[(Z)-2-cyano-2-(3,4-dimethoxy-phenyl)-vinyl]-thiophen-2-yl]-piperidin-4-yl ester (910 mg), produced during the production of Compound 1, was dissolved in chloroform (50 mL), and the solution was reacted with 4-hydroxypiperidine (233 mg) and triethylamine (233 mg) according to Production Step 4, to thereby yield the title compound (yield: 622 mg, 64%).

$^1$H-NMR ($CDCl_3$) δ:

7.37(1H,s),7.22(1H,d,J=4.4),7.13(1H,dd,J=8.5,2.2),7.04(1H,d,J =2.2),6.87(1H,d,J=8.5),6.05(1H,d,J=4.4),5.08-5.02 (1H, m), 3.94 (3H, s), 3.90 (3H, s), 3.77-3.70 (1H, m), 3.58-3.52 (2H, m), 3.31-3.27 (2H, m), 3.25 (2H, s), 2.87-2.82 (2H, m), 2.42-2.36(2H,m),2.43-2.35(2H,m),1.95-1.82(4H,m),1.70-1.60(2H,m)

Production of piperidin-1-yl-acetic acid 1-[5-[(Z)-2-cyano-2-(3,4-dimethoxy-phenyl)-vinyl]-thiophen-2-yl]-piperidin-4-yl ester hydrochloride (Compound 3)

6-mol/L Hydrochloric acid (352 μL), 2-propanol (25 mL), and ethanol (10 mL) were added to Compound 1 (950 mg), and the mixture was heated at about 80°C for dissolution. After allowing the solution to stand for cooling, the solvent was evaporated under reduced pressure, and the residue was recrystallized from 2-propanol-methanol, to thereby yield the title compound (yield: 869 mg, 85%).

$^1$H-NMR (DMSO-$d_6$) δ: 10.01(1H,br s),7.94(1H,s),7.42(1H,d,J=4.4),7.17(1H,d,J=2.2),7.09(1H,dd,J= 8.8,2.2),7.01(1H,d,J=8.8),6.32(1H,d,J=4.4),5.13-5.09 (1H,m),4.23(2H,br s),3.83(3H,s),3.78(3H,s),3.56-3.42(4H,m),3.40-3.30(4H,m),3.05-2.95(2H,m),2.08-2.02(2H,m), 1.85-1.65(6H,m)

Production of (4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester hydrochloride (Compound 4)

6-mol/L Hydrochloric acid (342 μL), 2-propanol (30 mL), and methanol (30 mL) were added to Compound 2 (956 mg), and the mixture was heated at about 80°C for dissolution. After allowing the solution to stand for cooling, the solvent was evaporated under reduced pressure, and the residue was recrystallized from 2-propanol, to thereby yield the title compound (yield: 947 mg, 93%).

$^1$H-NMR (DMSO-$d_6$) δ: 10.14(1H,br s),7.94(1H,s),7.42(1H,d,J=4.4),7.17(1H,d,J=2.0),7.09(1H,dd,J= 8.4,2.0),7.01(1H,d,J=8.4),6.32(1H,d,J=4.4),5. 5.07 (1H,m),4.29-4.23(2H,m),3.83(3H,s),3.78(3H,s),3.58-3.46(3H,m),3.40-3.26(6H,m),2.07-1.90(4H,m),1.85-1.65(4H,m)

Production of piperidin-1-yl-acetic acid 1-[5-[(Z)-2-cyano-2-(3,4-dimethoxy-phenyl)-vinyl]-thiophen-2-yl]-piperidin-4-yl ester methanesulfonate (Compound 5)

Methanesulfonic acid (137 μL), 2-propanol (20 mL), and methanol (5 mL) were added to Compound 1 (951 mg), and the mixture was heated at about 80°C for dissolution. After allowing the solution to stand for cooling, the solvent was evaporated under reduced pressure, and the residue was recrystallized from 2-propanol-methanol, to thereby yield the title compound (yield: 1,078 mg, 95%).

$^1$H-NMR (DMSO-$d_6$) δ: 9.78(1H,br s),7.93(1H,s),7.42(1H,d,J=4.4),7.17(1H,d,J=2.2),7.09(1H,dd,J= 8.8,2.2),7.01(1H,d,J=8.8),6.32(1H,d,J=4.4),5.13-5.09 (1H,m),4.24(2H,br s),3.83(3H,s),3.78(3H,s),3.55-3.40(4H,m),3.38-3.30(4H,m),3.06-2.94(2H,m),2.29(3H,s),2.08-2.02 (2H,m),1.85-1.65(6H,m)

Production of (4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester methanesulfonate (Compound 6)

Methanesulfonic acid (144 μL), 2-propanol (20 mL), and methanol (10 mL) were added to Compound 2 (1,029 mg), and the mixture was heated at about 80°C for dissolution. After allowing the solution to stand for cooling, the solvent was evaporated under reduced pressure, and the residue was recrystallized from 2-propanol-methanol, to thereby yield the title compound (yield: 696 mg, 57%).

$^1$H-NMR ($CDCl_3$) δ: 11.32(1H,br s),7.37(1H,s),7.22(1H,d,J=4.4),7.13(1H,dd,J=8.5,2.2),7.04(1H, d,J=2.2),6.88(1H,d,J=8.5),6.06(1H,d,J=4.4),5.1 5.12 (1H,m),4.29(1H,br s),3.95(3H,s),3.93(2H,br s),3.91(3H,s),3.65-3.52(4H,m),3.50-3.38(2H,m),3.33-3.25(2H,m),2.78(3H, s),2.48-2.30(2H,m),2.15-2.05(2H,m),2.00-1.87(4H,m),1.65-1.55(2H,m)

Production of azetidin-1-yl-acetic acid 1-[5-[(Z)-2-cyano-2-(3,4-dimethoxy-phenyl)-vinyl]-thiophen-2-yl]-piperidin-4-yl ester (Compound 7)

In a manner similar to that employed in the production of Compound 2, reaction was performed by use of azetidine and triethylamine according to Production Step 4, to thereby yield the title compound (yield: 74%).

$^1$H-NMR ($CDCl_3$) δ: 7.37(1H,s),7.22(1H,d,J=4.1),7.13(1H,dd,J=8.3,2.2),7.04(1H,d,J =2.2),6.87(1H,d,J=8.3),6.05(1H,d,J=4.1),5.07-4.98 (1H,m),3.95(3H,s),3.91(3H,s),3.59-3.50(2H,m),3.35(4H,t-like,J=7.1),3.31-3.22(2H,m),3.26(2H,s),2.14(2H,quint-like, J=7.1),2.08-1.97(2H,m),1.92-1.79(2H,m)

Production of azetidin-1-yl-acetic acid 1-[5-[(Z)-2-cyano-2-(3,4-dimethoxy-phenyl)-vinyl]-thiophen-2-yl]-piperidin-4-yl ester methanesulfonate (Compound 8)

Compound 7 (300 mg) was suspended in methanol (2 mL) and dissolved by adding 1-mol/L methanesulfonic acid (in methanol) (630 μL) to the suspension. The solvent was evaporated under reduced pressure, and ether was added the residue to form powder thereof, to thereby yield the title compound (yield: 345 mg, 95%).

MS (FAB) m/z : 468[M+H]$^+$

$^1$H-NMR ($CDCl_3$) δ: 11.80(1H,br s),7.37(1H,s),7.21(1H,d,J=4.1),7.13(1H,dd,J=8.5,2.2),7.03(1H, d,J=2.2),6.87(1H,d,J=8.5),6.06(1H,d,J=4.1),5.17-5.08 (1H,m),4.63-4.52(2H,m),4.15-4.02(4H,m),3.94(3H,s),3.90(3H,s),3.60-3.50(2H,m),3.34-3.24(2H,m),2.87-2.75(1H,m), 2.81(3H,s),2.56-2.41(1H,m),2.12-2.02(2H,m),1.97-1.85(2H,m)

Production of pyrrolidin-1-yl-acetic acid 1-[5-[(Z)-2-cyano-2-(3,4-dimethoxy-phenyl)-vinyl]-thiophen-2-yl]-piperidin-4-yl ester (Compound 9)

In a manner similar to that employed in the production of Compound 2, reaction was performed by use of pyrrolidine and triethylamine according to Production Step 4, to thereby yield the title compound (yield: 74%).

$^1$H-NMR ($CDCl_3$) δ: 7.37(1H,s),7.22(1H,d,J=4.4),7.13(1H,dd,J=8.3,2.2),7.04(1H,d,J =2.2),6.87(1H,d,J=8.3),6.05(1H,d,J=4.4),5.10-5.02 (1H, m), 3.95 (3H, s), 3.91 (3H, s), 3.62-3.50 (2H, m), 3.37 (2H, s), 3.32-3.22 (2H, m), 2.72-2.58 (4H, m), 2.10-1.99 (2H, m), 1.94-1.78(6H,m)

Production of pyrrolidin-1-yl-acetic acid 1-[5-[(Z)-2-cyano-2-(3,4-dimethoxy-phenyl)-vinyl]-thiophen-2-yl]-piperidin-4-yl ester methanesulfonate (Compound 10)

Compound 9 (300 mg) was suspended in methanol (3 mL) and dissolved by adding 1-mol/L methanesulfonic acid (in methanol) (620 μL) to the suspension. The solution was added to a mixture of ether (50 mL) and 2-propanol (10 mL) under stirring and cooling with ice, to thereby yield the title compound (yield: 342 mg, 95%).

MS (FAB) m/z:482 [M+H]$^+$

$^1$H-NMR ($CDCl_3$) δ: 11.58(1H,br s),7.37(1H,s),7.21(1H,d,J=4.4),7.12(1H,dd,J=8.5,2.2),7.04(1H, d,J=2.2),6.87(1H,d,J=8.5),6.06(1H,d,J=4.4),5.18-5.08 (1H,m),4.11(2H,s),4.05-3.91(2H,br m),3.94(3H,s),3.90(3H,s),3.61-3.49(2H,m),3.35-3.22(2H,m),3.18-3.01(2H,br),2.77 (3H,s),2.27-1.85(8H,m)

Production of (2-methyl-piperidin-1-yl)-acetic acid 1-[5-[(Z)-2-cyano-2-(3,4-dimethoxy-phenyl)-vinyl]-thiophen-2-yl]-piperidin-4-yl ester (Compound 11)

In a manner similar to that employed in the production of Compound 2, reaction was performed by use of 2-methyl-piperidine and triethylamine according to Production Step 4, to thereby yield the title compound (yield: 73%).

$^1$H-NMR ($CDCl_3$) δ: 7.36(1H,s),7.22(1H,d,J=4.4),7.13(1H,dd,J=8.3,2.2),7.04(1H,d,J =2.2),6.87(1H,d,J=8.3),6.05(1H,d,J=4.4),5.09-5.00 (1H,m),3.94(3H,s),3.90(3H,s),3.60-3.48(2H,m),3.41(2H,d,J=4.1),3.34-3.22(2H,m),2.91-2.82(1H,m),2.62-2.47(2H,m), 2.09-1.98(2H,m),1.81-1.80(2H,m),1.77-1.50(4H,m),1.38-1.21(2H,m),1.08(3H,d,J=6.3)

Production of (2-methyl-piperidin-1-yl)-acetic acid 1-[5-[(Z)-2-cyano-2-(3,4-dimethoxy-phenyl)-vinyl]-thiophen-2-yl]-piperidin-4-yl ester methanesulfonate (Compound 12)

Compound 11 (312 mg) was suspended in methanol (2 mL) and dissolved by adding 1-mol/L methanesulfonic acid (in methanol) (610 μL) to the suspension. The solvent was evaporated under reduced pressure, and ether was added the residue to form powder thereof, to thereby yield the title compound (yield: 320 mg, 86%).

MS(FAB) m/z: 510 [M+H]$^+$

$^1$H-NMR ($CDCl_3$) δ: 10.84(1H,br s),7.38(1H,s),7.22(1H,d,J=4.1),7.14(1H,dd,J=8.3,2.0),7.05(1H, d,J=2.0),6.88(1H,d,J=8.3),6.08(1H,d,J=4.1),5.15-5.06 (1H,m),4.24(1H,d,J=18.3),4.09(1H,d,J=18.3),3.94(3H,s),3.9 0(3H,s),3.75-3.64(1H,m),3.63-3.43(4H,m),3.35-3.24(2H, m),2.83(3H,s),2.15-2.03(3H,m),1.98-1.79(6H,m),1.62-1.48(1H,m),1.43(3H,d,J=6.3)

Production of (3-hydroxymethyl-piperidin-1-yl)-acetic acid 1-[5-[(Z)-2-cyano-2-(3,4-dimethoxy-phenyl)-vinyl]-thiophen-2-yl]-piperidin-4-yl ester (Compound 13)

In a manner similar to that employed in the production of Compound 2, reaction was performed by use of 3-hydroxymethyl-piperidine and triethylamine according to Production Step 4, to thereby yield the title compound (yield: 90%).

$^1$H-NMR ($CDCl_3$) δ:
7.36(1H,d,J=0.5),7.22(1H,dd,J=4.4,0.5),7.13(1H,dd,J=8.5,2.2), 7.04(1H,d,J=2.2),6.87(1H,d,J=8.5),6.05(1H,d,J=4.4), 5.09-5.00 (1H, m), 3.94 (3H, s), 3.90 (3H, s), 3.67-3.49 (4H, m), 3.33-3.22 (2H, m), 3.23 (2H, d, J=5.1), 2.90-2.80 (1H, m), 2.78-2.67(1H,m),2.39-2.17(2H,m),2.10-1.97(2H,m),1.93-1.55(7H,m),1.22-1.08(1H,m)

Production of (3-hydroxymethyl-piperidin-1-yl)-acetic acid 1-[5-[(Z)-2-cyano-2-(3,4-dimethoxy-phenyl)-vinyl]-thiophen-2-yl]-piperidin-4-yl ester methanesulfonate (Compound 14)

Compound 13 (330 mg) was suspended in methanol (3 mL) and dissolved by adding 1-mol/L methanesulfonic acid (in methanol) (630 μL) to the suspension. The solvent was evaporated under reduced pressure, and ether was added the residue to form powder thereof, to thereby yield the title compound (yield: 349 mg, 89%).

MS(FAB) m/z: 526[M+H]$^+$
$^1$H-NMR ($CDCl_3$) δ: 10.42(1H,br s),7.37(1H,s),7.21(1H,d,J=4.1),7.12(1H,dd,J=8.5,2.0),7.04(1H, d,J=2.0),6.87(1H,d,J=8.5),6.10(1H,d,J=4.1),5.19-5.10 (1H,m),4.12(1H,d,J=16.8),4.01(1H,d,J=16.8),3.94(3H,s),3.9 0(3H,s),3.81-3.65(2H,m),3.61-3.50(2H,m),3.47-3.38(1H, m),3.35-3.25(2H,m),3.20-3.05(4H,m),3.02-2.89(2H,m),2.80(3H,s),2.14-1.78(7H,m)

Production of azepan-1-yl-acetic acid 1-[5-[(Z)-2-cyano-2-(3,4-dimethoxy-phenyl)-vinyl]-thiophen-2-yl]-piperidin-4-yl ester (Compound 15)

In a manner similar to that employed in the production of Compound 2, reaction was performed by use of hexamethyleneimine and triethylamine according to Production Step 4, to thereby yield the title compound (yield: 75%).

$^1$H-NMR ($CDCl_3$) δ:
7.36(1H,s),7.22(1H,d,J=4.4),7.13(1H,dd,J=8.5,2.2),7.04(1H,d,J =2.2),6.87(1H,d,J=8.5),6.05(1H,d,J=4.4),5.08-5.00 (1H, m), 3.94 (3H, s), 3.90 (3H, s), 3.60-3.50 (2H, m), 3.41 (2H, s), 3.23-3.22 (2H, m), 2.82-2.71 (4H, m), 2.09-1.98 (2H, m), 1.92-1.80(2H,m),1.72-1.55(8H,m)

Production of azepan-1-yl-acetic acid 1-[5-[(Z)-2-cyano-2-(3,4-dimethoxy-phenyl)-vinyl]-thiophen-2-yl]-piperidin-4-yl ester methanesulfonate (Compound 16)

Compound 15 (300 mg) was suspended in methanol (2 mL) and dissolved by adding 1-mol/L methanesulfonic acid (in methanol) (590 μL) to the suspension. The solvent was evaporated under reduced pressure, and ether was added the residue to form powder thereof, to thereby yield the title compound (yield: 327 mg, 92%).

MS(FAB) m/z: 510[M+H]$^+$
$^1$H-NMR ($CDCl^3$) δ: 11.10(1H,br s),7.37(1H,s),7.22(1H,d,J=4.1),7.13(1H,dd,J=8.5,2.2),7.04(1H, d,J=2.2),6.88(1H,d,J=8.5),6.07(1H,d,J=4.1),5.17-5.09 (1H, m), 4.11 (2H, d, J=3.2), 3.94 (3H, s), 3.90 (3H, s), 3.67-3.50 (4H, m), 3.42-3.24 (4H, m), 2.82 (3H, s), 2.20-2.03 (4H, m), 1.98-1.82(6H,m),1.74-1.61(2H,m)

Production of azocan-1-yl-acetic acid 1-[5-[(Z)-2-cyano-2-(3,4-dimethoxy-phenyl)-vinyl]-thiophen-2-yl]-piperidin-4-yl ester (Compound 17)

In a manner similar to that employed in the production of Compound 2, reaction was performed by use of heptamethyleneimine and triethylamine according to Production Step 4, to thereby yield the title compound (yield: 71%).

$^1$H-NMR ($CDCl_3$) δ:
7.37(1H,s),7.22(1H,d,J=4.1),7.13(1H,dd,J=8.5,2.2),7.04(1H,d,J =2.2),6.87(1H,d,J=8.5),6.05(1H,d,J=4.1),5.08-5.00 (1H, m), 3.95 (3H, s), 3.91 (3H, s), 3.60-3.50 (2H, m), 3.39 (2H, s), 3.34-3.24 (2H, m), 2.77-2.65 (4H, br), 2.09-1.99 (2H, m), 1.92-1.80(2H,m),1.68-1.50(10H,m)

Production of azocan-1-yl-acetic acid 1-[5-[(Z)-2-cyano-2-(3,4-dimethoxy-phenyl)-vinyl]-thiophen-2-yl]-piperidin-4-yl ester methanesulfonate (Compound 18)

Compound 17 (300 mg) was suspended in methanol (2 mL) and dissolved by adding 1-mol/L methanesulfonic acid (in methanol) (570 μL) to the suspension. The solvent was evaporated under reduced pressure, and ether was added the residue to form powder thereof, to thereby yield the title compound (yield: 327 mg, 92%).

MS(FAB) m/z: 524[M+H]$^+$

$^1$H-NMR ($CDCl_3$) δ: 10.60(1H,br s),7.37(1H,s),7.22(1H,d,J=4.1),7.13(1H,dd,J=8.5,2.2),7.04(1H, d,J=2.2),6.87(1H,d,J=8.5),6.07(1H,d,J=4.1),5.18-5.09 (1H, m), 4.12 (2H, d, J=2.9), 3.94 (3H, s), 3.90 (3H, s), 3.69-3.50 (4H, m), 3.44-3.22 (4H, m), 2.80 (3H, s), 2.18-1.82 (10H, m), 1.78-1.53(4H,m)

Production of (4-methyl-piperazin-1-yl)-acetic acid 1-[5-[(Z)-2-cyano-2-(3,4-dimethoxy-phenyl)-vinyl]-thiophen-2-yl]-piperidin-4-yl ester (Compound 19)

In a manner similar to that employed in the production of Compound 2, reaction was performed by use of 4-methyl-piperazine and triethylamine according to Production Step 4, to thereby yield the title compound (yield: 69%).

$^1$H-NMR ($CDCl_3$) δ: 7.37(1H,s),7.22(1H,d,J=4.1),7.13(1H,dd,J=8.5,2.2),7.04(1H,d,J =2.2),6.88(1H,d,J=8.5),6.05(1H,d,J=4.1),5.08-5.00 (1H,m),3.95(3H,s),3.91(3H,s),3.60-3.50(2H,m),3.33-3.21(2H,m),3.24(2H,s),2.75-2.36(8H,br m),2.30(3H,s),2.09-1.99 (2H,m),1.92-1.81(2H,m)

Production of (4-methyl-piperazin-1-yl)-acetic acid 1-[5-[(Z)-2-cyano-2-(3,4-dimethoxy-phenyl)-vinyl]-thiophen-2-yl]-piperidin-4-yl ester methanesulfonate (Compound 20)

Compound 19 (270 mg) was suspended in methanol (2 mL) and dissolved by adding 1-mol/L methanesulfonic acid (in methanol) (530 μL) to the suspension. The solvent was evaporated under reduced pressure, and ether was added the residue to form powder thereof, to thereby yield the title compound (yield: 285 mg, 89%).

MS(FAB) m/z: 511[M+H]$^+$

$^1$H-NMR ($CDCl_3$) δ: 11.60-10.80(1H,br),7.37(1H,s),7.22(1H,d,J=4.1),7.13(1H,dd,J=8.5,2.2 ),7.04(1H,d,J=2.2),6.88(1H, d, J=8.5), 6.06 (1H, d, J=4.1), 5.10-5.00 (1H, m), 3.94 (3H, s), 3.90 (3H, s), 3.62-3.49 (4H, m), 3.41 (2H, s), 3.34-3.21 (2H, m), 3.18-3.00(6H,m),2.90(3H,s),2.82(3H,s),2.10-1.99(2H,m),1.93-1.81(2H,m)

Production of morpholin-4-yl-acetic acid 1-[5-[(Z)-2-cyano-2-(3,4-dimethoxy-phenyl)-vinyl]-thiophen-2-yl]-piperidin-4-yl ester (Compound 21)

In a manner similar to that employed in the production of Compound 2, reaction was performed by use of morpholine and triethylamine according to Production Step 4, to thereby yield the title compound (yield: 73%).

$^1$H-NMR ($CDCl_3$) δ: 7.37(1H,s),7.22(1H,d,J=4.1),7.13(1H,dd,J=8.5,2.2),7.04(1H,d,J =2.2),6.87(1H,d,J=8.5),6.05(1H,d,J=4.1),5.10-5.00 (1H, m), 3.94 (3H, s), 3.90 (3H, s), 3.80-3.70 (4H, m), 3.60-3.50 (2H, m), 3.34-3.20 (2H, m), 3.24 (2H, s), 2.65-2.54 (4H, m), 2.10-1.98(2H,m),1.93-1.80(2H,m)

Production of morpholin-4-yl-acetic acid 1-[5-[(Z)-2-cyano-2-(3,4-dimethoxy-phenyl)-vinyl]-thiophen-2-yl]-piperidin-4-yl ester methanesulfonate (Compound 22)

Compound 21 (300 mg) was suspended in methanol (2 mL) and dissolved by adding 1-mol/L methanesulfonic acid (in methanol) (600 μL) to the suspension. The solution was added to ether (50 mL) under stirring and cooling with ice, to thereby yield the title compound (yield: 340 mg, 95%).

MS(FAB) m/z: 498[M+H]$^+$

$^1$H-NMR ($CDCl_3$) δ: 13.00-11.00(1H,br),7.37(1H,s),7.21(1H,d,J=4.1),7.12(1H,dd,J=8.5,2.2 ),7.04(1H,d,J=2.2),6.87(1H, d, J=8.5), 6.06 (1H, d, J=4.1), 5.19-5.10 (1H, m), 4.19-3.97 (4H, m), 4.04 (2H, s), 3.94 (3H, s), 3.90 (3H, s), 3.80-3.62 (2H, br), 3.58-3.49(2H,m),3.38-3.17(4H,m),2.78(3H,s),2.13-2.03(2H,m),1.98-1.86(2H,m)

Example 2: Effect of overcoming anticancer agent resistance *in vivo* (oral administration)

To an inguinal region of each of 6-week-old BALB/c male nude mice (5 mice/group), BCRP-gene-transfected human colon cancer HCT116 cells (HCT116/BCRP cells) (obtained from Dr. Yoshikazu Sugimoto, The Cancer Chemotherapy Center of Japanese Foundation for Cancer Research) were subcutaneously transplanted ($2 \times 10^6$ cells/0.1 mL/mouse). When the tumor volume as estimated from $(1/2)ab^2$ (a: longer tumor diameter, b: shorter tumor diameter) reached about 100 to 150 $mm^3$, each of the compounds of the present invention shown in Tables 1 to 4 and a positive control compound (i.e., Compound 14 ((Z)-2-(3,4-dimethoxy-phenyl)-3-{5-[4-(2-hydroxy-ethyl)-piperazin-1-yl]-thiophen-2-yl}-acrylonitrile hydrochloride)), disclosed in Patent Document 3 (WO 2006/106778)) (hereinafter referred to as Earlier Application Compound 14) was orally administered once a day for nine days (9 times in total). Separately, CPT-11 (45 mg/kg/day) was intravenously administered once a day to the mice on day 1, day 5, and day 9 (3 times in total). The dose of each of the compounds of the present invention was adjusted to 0.20 or 0.40 mmol/kg/day, and that of the positive control compound was adjusted to 0.23 or 0.46 mmol/kg/day. For administration, Compound 3 or 5 of the present invention was dissolved in 5% glucose solution, and Compound 4 or 6 was dissolved in Britton-Robinson buffer. Earlier Application Compound 14 was dissolved in 0.1% Tween 80 solution, and CPT-11 was dissolved in physiological saline. A solvent was solely administered to a control group. On day 21 from start of administration, a tumor was extirpated from each mouse and weighed, and the tumor growth inhibition ratio IR (%) was derived from the following equation:

$$\text{Tumor growth inhibition ratio IR (\%)} = (1 - \text{average tumor weight of each administration group/average tumor weight of control group}) \times 100.$$

The results are shown in Tables 1 to 4.

As is clear from Tables 1 to 3, the acrylonitrile derivatives of the present invention potently inhibit BCRP also in vivo, exhibiting a remarkably excellent effect of overcoming resistance to anticancer agent.

As compared with Earlier Application Compound 14 (see; Table 4), which was previously confirmed to exhibit an excellent effect of overcoming resistance to anticancer agent *in vivo*, the compounds of the present invention were found to exhibit further more excellent effect of overcoming resistance to an anticancer agent.

[Table 1]

| Compound | Dose (mg/kg/day) | | Tumor weight (g) Mean±S.D. | IR (%) |
|---|---|---|---|---|
| | Compound | CPT-11 | | |
| 3 | 108.1 | 45 | 0.73±0.07*** | 51.9 |
| | 216.3 | 45 | 0.65±0.11*** | 54.7 |
| Solvent | 0 | 45 | 1.19±0.16 | 21.3 |
| | 0 | 0 | 1.51±0.19 | |

***P<0.001; significant difference with respect to solvent + CPT-11 (HCT116/BCRP) (Dunnett's test)

[Table 2]

| Compound | Dose (mg/kg/day) | | Tumor weight (g) Mean±S.D. | IR (%) |
|---|---|---|---|---|
| | Compound | CPT-11 | | |
| 5 | 120.3 | 45 | 0.73±0.12*** | 50.9 |
| | 240.5 | 45 | 0.65±0.16*** | 55.9 |
| Solvent | 0 | 45 | 1.13±0.07 | 23.4 |
| | 0 | 0 | 1.48±0.19 | |

***P<0.001; significant difference with respect to solvent + CPT-11 (HCT116/BCRP) (Dunnett's test)

[Table 3]

| Compound | Dose (mg/kg/day) | | Tumor weight (g) Mean±S.D. | IR (%) |
|---|---|---|---|---|
| | Compound | CPT-11 | | |
| 4 | 111.4 | 45 | 0.89±0.09*** | 31.8 |
| | 222.8 | 45 | 0.70±0.11*** | 46.4 |
| 6 | 123.5 | 45 | 0.82±0.04*** | 36.9 |
| | 247.0 | 45 | 0.76±0.09*** | 41.8 |
| Solvent | 0 | 45 | 1.22±0.12 | 6.0 |
| | 0 | 0 | 1.30±0.22 | |

***P<0.001; significant difference with respect to solvent + CPT-11 (HCT116/BCRP) (Dunnett's test)

[Table 4]

| Compound | Dose (mg/kg/day) | | Tumor weight (g) Mean±S.D. | IR (%) |
|---|---|---|---|---|
| | Compound | CPT-11 | | |
| EAC 14 | 100 | 45 | 1.04±0.17 | 23.9 |
| | 200 | 45 | 0.88±0.14* | 35.6 |
| Solvent | 0 | 45 | 1.16±0.13 | 15.3 |
| | 0 | 0 | 1.37±0.22 | |

*P<0.05; significant difference with respect to solvent + CPT-11 (HCT116/BCRP) (Dunnett's test)
EAC: Earlier Application Compound

Example 3: Solubility

Each (10 mg) of the compounds of the present invention and Earlier Application Compound 14 shown in Table 5 was weighed and placed in a sealable container, and water or 5% glucose solution was added to the container in such an amount as to adjust the final concentration to 100 mg/mL. The contents of the container were stirred at room temperature for about 30 minutes, followed by centrifugation. The compound concentration of the supernatant recovered through centrifugation was quantified through high-performance liquid chromatography. Table 5 shows the results. Some compounds falling within the scope of the present invention (Compounds 5, 12, 14, and 20), and Earlier Application Compound 14 were analyzed in terms of solubility in physiological saline, in a manner similar to that employed in quantification of the compound concentration of the supernatant, except that physiological saline was used as a solvent instead of water or glucose.
As compared with Earlier Application Compound 14, the compounds of the present invention (Compounds 5, 8, 10, 12, 14, 16, 18, 20, and 22) exhibited remarkably high solubility in water and 5% glucose solution. Earlier Application Compound 14 was found to have poor solubility in physiological saline (<1 mg/mL), whereas the compounds of the present invention (Compounds 5, 12, 14, and 20) were found to have high solubility (≥100 mg/mL) not depending on the composition of the solvent.
Thus, the compounds falling within the scope of the present invention were found to have advantageous **characteristics in that** the compounds have considerably high solubility, and that use thereof is not limited by the composition of transfusion, in particular, upon administration to patients.
[Table 5]

| Solvent | Water | 5% Glucose |
|---|---|---|
| EAC 14 | 6.6 mg/mL | 6.6 mg/mL |
| Compound 5 | ≥100 mg/mL | ≥100 mg/mL |
| Compound 8 | ≥100 mg/mL | ≥100 mg/mL |
| Compound 10 | ≥100 mg/mL | ≥100 mg/mL |
| Compound 12 | ≥100 mg/mL | ≥100 mg/mL |
| Compound 14 | ≥100 mg/mL | ≥100 mg/mL |
| Compound 16 | ≥100 mg/mL | ≥100 mg/mL |
| Compound 18 | ≥100 mg/mL | ≥100 mg/mL |
| Compound 20 | ≥100 mg/mL | ≥100 mg/mL |
| Compound 22 | ≥100 mg/mL | ≥100 mg/mL |
| EAC: Earlier Application Compound | | |

Example 4:

The following ingredients were mixed and tabletted.

[Table 6]

| | |
|---|---|
| Compound 5 | 100 mg |
| Lactose | 100 mg |
| Potato starch | 39 mg |
| Microcrystalline cellulose | 30 mg |
| Synthetic aluminum silicate | 30 mg |
| Calcium stearate | 1 mg |
| Total (one tablet) | 300 mg |

## Claims

1. An acrylonitrile derivative represented by formula (1) :

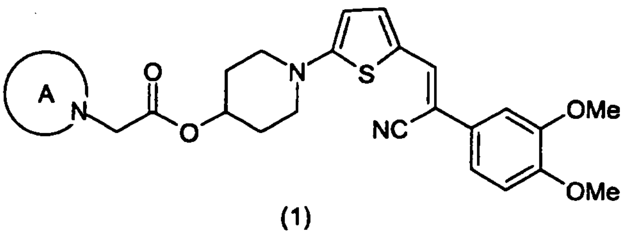

(1) (E or Z)

(wherein A represents an optionally substituted 3- to 8-membered heterocyclic ring) or a salt thereof.

2. An acrylonitrile derivative or a salt thereof according to claim 1, wherein A is one species selected from among the following formulas (3) to (9)

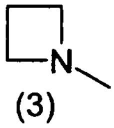
(3)
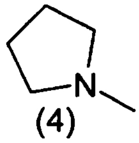
(4)
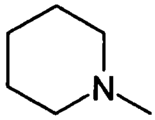
(5)
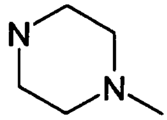
(6)

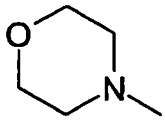
(7)
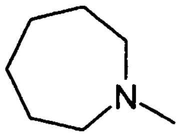
(8)
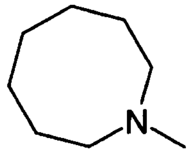
(9)

which may have a substituent.

**3.** An acrylonitrile derivative selected from the following:

piperidin-1-yl-acetic acid 1-[5-[(Z)-2-cyano-2-(3,4-dimethoxy-phenyl)-vinyl]-thiophen-2-yl]-piperidin-4-yl ester,
(4-hydroxy-piperidin-1-yl)-acetic acid 1-[5-[(Z)-2-cyano-2-(3,4-dimethoxy-phenyl)-vinyl]-thiophen-2-yl]-piperidin-4-yl ester,
azetidin-1-yl-acetic acid 1-[5-[(Z)-2-cyano-2-(3,4-dimethoxy-phenyl)-vinyl]-thiophen-2-yl]-piperidin-4-yl ester,
pyrrolidin-1-yl-acetic acid 1-[5-[(Z)-2-cyano-2-(3,4-dimethoxy-phenyl)-vinyl]-thiophen-2-yl]-piperidin-4-yl ester,
(2-methyl-piperidin-1-yl)-acetic acid 1-[5-[(Z)-2-cyano-2-(3,4-dimethoxy-phenyl)-vinyl]-thiophen-2-yl]-piperidin-4-yl ester,
(3-hydroxymethyl-piperidin-1-yl)-acetic acid 1-[5-[(Z)-2-cyano-2-(3,4-dimethoxy-phenyl)-vinyl]-thiophen-2-yl]-piperidin-4-yl ester,
azepan-1-yl-acetic acid 1-[5-[(Z)-2-cyano-2-(3,4-dimethoxy-phenyl)-vinyl]-thiophen-2-yl]-piperidin-4-yl ester,
azocan-1-yl-acetic acid 1-[5-[(Z)-2-cyano-2-(3,4-dimethoxy-phenyl)-vinyl]-thiophen-2-yl]-piperidin-4-yl ester,
(4-methyl-piperazin-1-yl)-acetic acid 1-[5-[(Z)-2-cyano-2-(3,4-dimethoxy-phenyl)-vinyl]-thiophen-2-yl]-piperidin-4-yl ester, and
morpholin-4-yl-acetic acid 1-[5-[(Z)-2-cyano-2-(3,4-dimethoxy-phenyl)-vinyl]-thiophen-2-yl]-piperidin-4-yl ester,
or a salt thereof.

**4.** A drug comprising, as an active ingredient, an acrylonitrile derivative or a salt thereof as recited in any one of claims 1 to 3.

**5.** A BCRP inhibitor comprising, as an active ingredient, an acrylonitrile derivative or a salt thereof as recited in any one of claims 1 to 3.

**6.** An agent for overcoming anticancer agent resistance or an agent for potentiating anticancer agent effect, which agent comprises, as an active ingredient, an acrylonitrile derivative or a salt thereof as recited in any one of claims 1 to 3.

**7.** A pharmaceutical composition comprising an acrylonitrile derivative or a salt thereof as recited in any one of claims 1 to 3, and a pharmaceutically acceptable carrier.

**8.** An anticancer agent composition comprising an acrylonitrile derivative or a salt thereof as recited in any one of claims 1 to 3, and an anticancer agent which serves as a BCRP substrate.

**9.** Use of an acrylonitrile derivative or a salt thereof as recited in any one of claims 1 to 3 for producing an agent for overcoming anticancer agent resistance or an agent for potentiating anticancer agent effect.

**10.** Use of an acrylonitrile derivative or a salt thereof as recited in any one of claims 1 to 3 for producing an anticancer agent composition comprising an anticancer agent which serves as a BCRP substrate.

**11.** A method for treatment of cancer which has acquired drug resistance by the mediation of BCRP, the method comprising administering an acrylonitrile derivative or a salt thereof as recited in any one of claims 1 to 3.

**12.** A method for inhibiting BCRP, the method comprising administering an acrylonitrile derivative or a salt thereof as recited in any one of claims 1 to 3.

**13.** A method for overcoming anticancer agent resistance or for potentiating anticancer agent effect, the method comprising administering an acrylonitrile derivative or a salt thereof as recited in any one of claims 1 to 3.

**14.** A method for treatment of cancer, the method comprising administering an acrylonitrile derivative or a salt as recited in any one of claims 1 to 3, and an anticancer agent which serves as a BCRP substrate.

**INTERNATIONAL SEARCH REPORT**

International application No.
PCT/JP2008/003557

A. CLASSIFICATION OF SUBJECT MATTER
*C07D409/04*(2006.01)i, *A61K31/4535*(2006.01)i, *A61K31/454*(2006.01)i, *A61K31/4545*(2006.01)i, *A61K31/496*(2006.01)i, *A61K31/5377*(2006.01)i, *A61K31/55*(2006.01)i, *A61P35/00*(2006.01)i, *A61P43/00*(2006.01)i,

According to International Patent Classification (IPC) or to both national classification and IPC

B. FIELDS SEARCHED

Minimum documentation searched (classification system followed by classification symbols)
C07D409/04-C07D409/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
REGISTRY/CAplus/BIOSIS/MEDLINE(STN), WPI

C. DOCUMENTS CONSIDERED TO BE RELEVANT

| Category* | Citation of document, with indication, where appropriate, of the relevant passages | Relevant to claim No. |
|---|---|---|
| X | WO 2006/106778 A1 (Yakult Honsha Co., Ltd.), 12 October, 2006 (12.10.06), & EP 1864972 A1 & KR 10-2008-0006543 A & CN 101166719 A | 1-10 |

☐ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>02 March, 2009 (02.03.09) | Date of mailing of the international search report<br>10 March, 2009 (10.03.09) |
| Name and mailing address of the ISA/<br>Japanese Patent Office<br><br>Facsimile No. | Authorized officer<br><br>Telephone No. |

Form PCT/ISA/210 (second sheet) (April 2007)

**INTERNATIONAL SEARCH REPORT**

International application No.
PCT/JP2008/003557

Continuation of A. CLASSIFICATION OF SUBJECT MATTER
(International Patent Classification (IPC))

*C07D409/14*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2008/003557

**Box No. II Observations where certain claims were found unsearchable (Continuation of item 2 of first sheet)**

This international search report has not been established in respect of certain claims under Article 17(2)(a) for the following reasons:

1. [X] Claims Nos.: 11-14
because they relate to subject matter not required to be searched by this Authority, namely:
Claims 11 to 14 include the methods for treatment of the human body or animal body by surgery or therapy and for diagnosis of the same.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004069233 A
- WO 2004069243 A
- WO 2006106778 A


### Non-patent literature cited in the description


- *Proc. Natl. Acad. Sci. USA,* 1998, vol. 95, 15665-15670
- *Cancer Res.,* 1999, vol. 59, 5938-5946
- *J. Clin. Oncol.,* 2002, vol. 20, 2943-2950
- *Mol. Cancer Ther.,* 2002, vol. 1, 417-425
- *Mol. Cancer Ther.,* 2003, vol. 2, 105-112
- *Int. J. Cancer,* 2004, vol. 108, 146-151